# FASCICULE DE BREVET EUROPEEN

(11) **EP 0 527 696 B1**
(45) Date de publication et mention de la délivrance du brevet: **28.06.1995**
(21) Numéro de dépôt: 92420278.1
(22) Date de dépôt: 14.08.1992
(51) Int. Cl.: A61M 1/34, A61M 1/16

(54) **Rein artificiel et procédé de commande**
Künstliche Niere und Steuerungsverfahren
Artificial kidney and control method therefor

(30) Priorité: 14.08.1991 FR 9110463
(43) Date de publication de la demande: 17.02.1993
(73) Titulaire: HOSPAL INDUSTRIE, F-69883 Meyzieu Cédex (FR)
(72) Inventeur: Chevallet, Jacques, F-69360 Serezin Du Rhone (FR)

(56) Documents cités:
- EP-A- 0 122 604
- EP-A- 0 212 127

## Description

La présente invention a pour objet un rein artificiel, et, plus particulièrement un rein artificiel destiné au traitement des personnes privées temporairement, à la suite d'un accident ou d'une opération chirurgicale, de l'usage de leurs reins.

Compte tenu de l'état d'affaiblissement général dans lequel ils se trouvent, ces patients ne peuvent être soumis aux traitements intensifs que subissent les patients souffrant d'une insuffisance rénale définitive : une séance classique d'hémodialyse ou d'hémofiltration bihebdomadaire de quatre heures, par la rapide modification des équilibres liquidiens internes qu'elle provoque, a en effet pour corollaire une sollicitation intense du système cardio-vasculaire que des patients sortant du bloc opératoire ne pourraient, en règle générale, supporter.

Pour épurer le sang de ces patients et leur faire éliminer une partie de l'eau qui s'accumule dans leurs tissus, on recourt donc à des traitements peu intenses mais continus, qui sont à la fois bien tolérés par l'organisme car sans à-coups, et supportables par des personnes qui sont hors d'état de se déplacer.

De façon classique, les patients mentionnés plus haut sont soumis à deux types de traitement, l'hémofiltration et l'hémodialyse continues.

Le principe de l'hémofiltration consiste à retirer du sang, par ultrafiltration au travers d'une membrane semi-perméable, une partie de son eau plasmatique chargée en impuretés. Le moteur du transfert est la différence des pressions de part et d'autre de la membrane. Un liquide de substitution peut être perfusé simultanément au patient, en quantité généralement inférieure à la quantité de filtrat soutirée, la différence correspondant au poids qu'il apparaît souhaitable de lui faire perdre. Le débit d'ultrafiltration, c'est-à-dire aussi l'efficacité du traitement par hémofiltration, est limité à la fois par les caractéristiques du filtre (nature et surface de la membrane, essentiellement) et par le débit du sang dans le filtre, ce débit étant relativement faible dans les traitements continus (4 à 12 litres/heure contre 12 à 21 litres/heure dans le traitement des patients ayant définitivement perdu l'usage de leurs reins).

Dans le traitement par hémodialyse, les impuretés du sang ne sont pas entrainées par convection dans un flux d'eau plasmatique traversant une membrane semi-perméable comme c'est donc le cas en hémofiltration, mais elles migrent par diffusion au travers d'une membrane semi-perméable dont la face non baignée par le sang est irriguée par un flux de liquide de dialyse, dépourvu des substances à épurer. Le moteur du transfert est alors la différence des concentrations de part et d'autre de la membrane.

Pour un même filtre du type à haute perméabilité, et pour un même débit de sang, il est possible d'obtenir, en choisissant convenablement le débit du liquide de dialyse, une épuration beaucoup plus efficace des impuretés de faible poids moléculaire par hémodialyse que par hémofiltration. En revanche, l'hémofiltration permet, outre l'élimination d'une partie de l'eau accumulée dans les tissus, celle d'impuretés de poids moléculaire élevé qui ne migrent pas ou peu par diffusion.

De ce qui précède, on comprend que, pour épurer efficacement le sang d'un patient ayant perdu l'usage de ses reins, il faut pouvoir le soumettre à la fois à un traitement par hémofiltration et par hémodialyse.

Le brevet européen 256 956 décrit un rein artificiel permettant la mise en oeuvre alternative ou simultanée de ces traitements. Ce rein comporte un échangeur comprenant deux compartiments séparés par une membrane semi-perméable. Un premier compartiment est connecté à un circuit pour la circulation extracorporelle de sang. Le second compartiment a une entrée pouvant être connectée, par l'intermédiaire d'une vanne trois voies, à un premier réservoir pour liquide de dialyse stérile, et une sortie connectée à un second réservoir destiné à recueillir le liquide de dialyse usé et le filtrat du sang. La vanne trois voies permet également de connecter le premier réservoir au circuit sang, en aval de l'échangeur, le liquide de dialyse stérile pouvant être utilisé comme liquide de substitution injectable. La circulation du liquide stérile peut être spontanée ou être provoquée par des pompes, de même que l'extraction du filtrat ou du liquide de dialyse usé.

Avec ce rein artificiel, la sélection du mode de traitement est effectuée manuellement, par manipulation de la vanne trois voies, sur prescription du médecin.

L'invention a pour but la réalisation d'un rein artificiel de ce type, mais sur lequel la sélection du mode de traitement se fait automatiquement pour assurer une épuration maximale du patient, compte tenu des prescriptions du médecin.

Pour atteindre ce but, on prévoit, conformément à l'invention un rein artificile comprenant :
- un circuit pour circulation extracorporelle de sang connectable à une source de liquide stérile,
- un échangeur ayant deux compartiments séparés par une membrane semi-perméable, un premier compartiment étant connecté au circuit pour circulation extracorporelle de sang, un second compartiment ayant une entrée connectable à une source de liquide stérile,
- des moyens pour créer une pression transmembranaire positive entre le premier et le second compartiment de l'échangeur, caractérisé en ce qu'il comprend des moyens pour mesurer une valeur représentative de la pression transmembranaire dans l'échangeur et des moyens de commande pour commander la connexion d'une source de liquide stérile à l'entrée du second compartiment de l'échangeur quand cette valeur mesurée est supérieure à une valeur de seuil prédéterminée.

Selon une caractéristique de l'invention, les moyens de commande sont prévus en outre pour commander la connexion d'une source de liquide stérile au circuit pour circulation extracorporelle de sang quand la valeur mesurée est inférieure à une deuxième valeur de seuil prédéterminée, cette deuxième valeur de seuil étant inférieure à la première valeur de seuil.

Selon une autre caractéristique de l'invention, les moyens de commande sont prévus en outre pour commander alternativement, selon une séquence temporelle, la connexion d'une source de liquide stérile à l'entrée du second compartiment de l'échangeur et la connexion de la source de liquide stérile au circuit pour circulation extracorporelle de sang.

L'invention a en outre pour objet un procédé de commande d'un rein artificiel consistant à :
- mesurer une valeur représentative de la pression transmembranaire dans l'échangeur,
- comparer cette valeur à une valeur de seuil prédéterminée, et
- commander la connexion d'une source de liquide stérile à l'entrée du second compartiment de l'échangeur quand la valeur mesurée est supérieure à la valeur de seuil prédéterminée.

Selon une première variante de l'invention, le procédé consiste en outre à :
- comparer la valeur mesurée représentative de la pression transmembranaire à une deuxième valeur de seuil prédéterminée, cette deuxième valeur de seuil étant inférieure à la première valeur de seuil, et
- commander la connexion d'une source de liquide stérile au circuit pour circulation extracorporelle de sang quand la valeur mesurée est inférieure à la deuxième valeur de seuil.

Selon une seconde variante de l'invention, le procédé consiste en outre, à partir du moment où la valeur mesurée a atteint la valeur de seuil prédéterminée, à commander alternativement, selon une séquence temporelle prédéterminée, la connexion d'une source de liquide à l'entrée du second compartiment de l'échangeur et au circuit pour circulation extracorporelle de sang.

D'autres caractéristiques et avantages de l'invention apparaîtront à la lecture de la description qui suit. On se reportera aux dessins annexés sur lesquels :
la figure 1 est un schéma simplifié d'un premier mode de réalisation du rein artificiel selon l'invention, et
la figure 2 est un schéma simplifié d'un second mode de réalisation du rein artificiel selon l'invention.

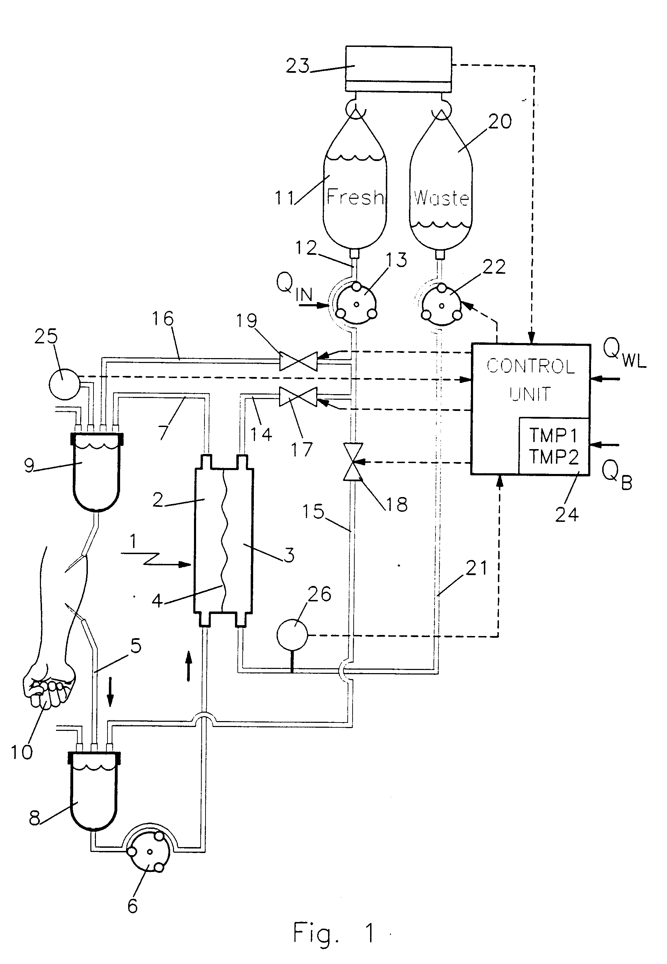

Sur la figure 1, on voit que le rein artificiel selon l'invention comporte un échangeur 1 comprenant deux compartiments 2, 3 séparés par une membrane semi-perméable 4. Le compartiment 2 est connecté à un circuit de circulation extracorporelle de sang comprenant une canalisation amont 5 sur laquelle est disposée une pompe de circulation 6, et une canalisation aval 7. Ces canalisations 5, 7 sont munies chacune d'un piège à bulles 8, respectivement 9 et, à leur extrémité libre, d'une aiguille ou d'un connecteur pour cathéter, pour leur raccordement au circuit vasculaire d'un patient 10.

Un premier réservoir 11 pour liquide de dialyse/substitution stérile est relié, par l'intermédiaire d'un tronçon de canalisation commune 12 sur lequel est disposée une pompe de circulation 13, à trois canalisations 14, 15, 16 connectées respectivement à une entrée du compartiment 3 de l'échangeur 1, au piège à bulles amont 8 et au piège à bulles aval 9. Un moyen d'occlusion 17, 18, 19, tel qu'un clamp électromagnétique est disposé sur chacune des canalisations 14, 15, 16 pour permettre de relier sélectivement le réservoir 11 à l'une ou l'autre d'entre elles.

Un deuxième réservoir 20 pour le recueil du liquide de dialyse usé (filtrat du sang et/ou liquide de dialyse usé) est relié à une sortie du compartiment 3 de l'échangeur 1, par une canalisation 21 sur laquelle est disposée un moyen pour créer une dépression variable dans le compartiment 3, constitué par une pompe d'extraction 22.

Les deux réservoirs 11, 20 sont assujettis à un moyen 23 pour faire le bilan des liquides stérile et usé, tel qu'une balance, dont le signal est fourni à une unité de commande 24. Cette unité de commande reçoit par ailleurs les signaux fournis par deux capteurs de pression 25, 26 disposés respectivement sur le circuit de sang en aval de l'échangeur 1 et sur le circuit de liquide usé, entre l'échangeur 1 et la pompe d'extraction 22. A partir de ces mesures de poids et de pression et d'une consigne de débit de perte de poids Q_{W L} correspondant à l'équilibre ou à un déséquilibre souhaité des liquides stériles et usés, l'unité de commande 24 pilote, selon un mode de fonctionnement expliqué plus loin, l'ouverture et la fermeture des clamps 17, 19 ainsi que le débit de la pompe 22, le débit de la pompe 13 étant réglé à une valeur fixe par ailleurs.

Avant de décrire le fonctionnement de ce rein, il est nécessaire de compléter brièvement ce qui a été rappelé plus haut à propos de l'hémofiltration, à savoir que le débit de l'ultrafiltrat dans un échangeur est fonction de la différence des pressions (ou pression transmembranaire) qui règnent dans les deux compartiments de l'échangeur et que ce débit est limité à la fois par la surface et la nature de la membrane, qui peut être plus ou moins perméable, et par le débit du sang dans l'échangeur : on ne peut extraire du sang plus du tiers de son eau plasmatique, sauf à risquer de colmater l'échangeur avec un sang trop concentré. A ces deux facteurs de limitation s'en ajoute un troisième, qui tient à la nature même du sang. On remarque en effet que, pour une membrane de nature donnée et pour un débit de sang donné dans l'échangeur, à partir d'une certaine valeur de la pression transmembranaire, le débit d'ultrafiltration tend rapidement vers une valeur sensiblement constante. Ce phénomène est dû au fait que, sous l'effet de la convection du plasma causée par la pression transmembranaire, les protéines plasmatiques s'accumulent au voisinage de la membrane et en engendrent une pression osmotique qui limite la pression transmembranaire.

Ce rappel étant fait, le principe de fonctionnement du rein arficiel selon l'invention est le suivant : un patient venant de perdre l'usage de ses reins est traité a priori par hémofiltration. Le liquide stérile contenu dans le réservoir 11 est alors utilisé comme liquide de substitution et lui est perfusé. Si maintenant, en début ou en cours de séance, parce qu'il trouve par exemple que la vitesse d'épuration n'est pas suffisante, le médecin, par une commande qui sera expliquée plus loin en détail, provoque l'augmentation de la pression transmembranaire dans l'échangeur au-delà d'une valeur de seuil correspondant sensiblement au débit d'ultrafiltration maximal, le rein se met automatiquement à fonctionner en mode hémodialyse, le liquide stérile du réservoir 11 étant alors utilisé comme liquide de dialyse et circulant dans l'échangeur 1.

De façon détaillée, le rein artificiel selon l'invention fonctionne de la façon suivante : avant le début de la séance de traitement, un opérateur met en mémoire dans l'unité de commande une consigne de débit de perte de poids Q_{WL} souhaité (égale, par définition, à la différence souhaitée à tout instant entre le débit d'ultrafiltration et le débit de liquide de substitution) et une consigne de débit de sang Q_{B} souhaité, prescrites par le médecin. A cette valeur de débit de sang Q_{B}, l'unité de commande 24 associe automatiquement, selon une loi de correspondance préalablement mise en mémoire, deux valeurs de seuil supérieure et inférieure de pression transmembranaire TMP₁ et TMP₂, spécifiques à l'échangeur 1 utilisé, TMP₁ correspondant avantageusement à la pression transmembranaire au-delà de laquelle le débit d'ultrafiltration reste sensiblement constant au débit de sang Q_{B} choisi.

Le rinçage et le remplissage initiaux des canalisations ayant été effectués et le circuit de circulation extracorporelle de sang ayant été raccordé au circuit vasculaire du patient 10, les pompes 6 et 13 sont réglées à des débits constants.

Deux cas peuvent se présenter alors, selon que le sang du patient 10 doit être soumis à une épuration modérée ou plus intense. Dans le premier cas, le débit Q_{IN} de la pompe de liquide stérile 13 est choisi inférieur au débit d'ultrafiltration correspondant à TMP₁ diminué du débit de perte de poids Q_{WL}. Le résultat de la comparaison effectuée par l'unité de commande 24 entre TMP₁ et la pression transmembranaire dans l'échangeur 1 mesurée à partir des informations fournies par les capteurs de pression 25, 26, indiquant la supériorité de TMP₁, l'unité de commande maintient les clamps 17, 18 fermés et le clamps 19 ouvert : le rein fonctionne en mode hémofiltration, le liquide stérile contenu dans le réservoir 11 est perfusé au patient tandis que le filtrat extrait dans l'échangeur 1 grâce à la dépression créée dans le compartiment 3 par la pompe 22 remplit le deuxième réservoir 20. La pompe 22 est pilotée en permanence par l'unité de contrôle 24 en fonction des informations fournies par la balance 23 pour que le débit de perte de poids réel soit égal au débit de perte de poids souhaité Q_{WL}.

Si le médecin juge que la vitesse d'épuration du sang du patient 10 est insuffisante avec le débit Q_{IN} choisi initialement, ce débit doit être augmenté. Si ce débit Q_{IN} est augmenté jusqu'à devenir supérieur ou égal au débit d'ultrafiltration correspondant à TMP₁ diminué du débit de perte de poids Q_{WL}, la pression transmembranaire mesurée dans l'échangeur 1 devient supérieure ou égale à TMP₁ et l'unité de commande 24 commande alors la fermeture du clamp 19 et l'ouverture simultanée du clamp 17, le clamp 18 restant fermé. Le rein fonctionne alors en mode "hémodiafiltration" dans lequel les effets de la dialyse se combinent à ceux de l'ultrafiltration, le liquide stérile contenu dans le premier réservoir 11 s'écoulant alors dans le compartiment 3 de l'échangeur 1, où continue par ailleurs de migrer par ultrafiltration de l'eau plasmatique. L'ouverture du clamp 17 et l'écoulement de liquide qui en résulte dans le compartiment 3 de l'échangeur 1 provoque une chute de la pression transmembranaire. Quand la pression transmembranaire atteint le niveau ce TMP₂, l'unité de commande 24 inverse la position des clamps 17, 19 et le rein repasse en mode hémofiltration jusqu'à ce que la pression transmembranaire atteigne de nouveau le niveau de TMP₁, et ainsi de suite.

Dans le rein selon l'invention, l'épuration rapide du sang d'un patient se fait donc par l'alternance de phases d'hémofiltration et d'hémodiafiltration, la fréquence de cette alternance pouvant être réglée par le choix de TMP₂.

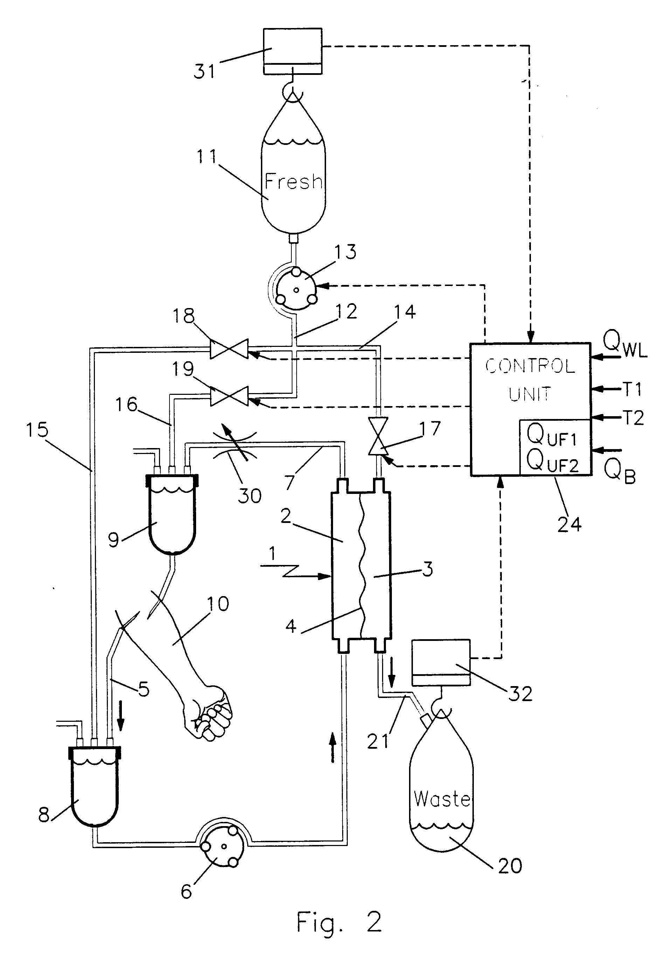

La figure 2 représente un second mode de réalisation de l'invention ayant en commun avec le précédent son circuit pour circulation extracorporelle de sang et ses circuits de liquides stérile et usé. Il s'en différencie en ce que le moyen pour créer une pression transmembranaire variable dans l'échangeur 1 est constitué par un organe de retreint réglable 30 disposé sur la canalisation aval 7 du circuit pour circulation extracorporelle de sang. La pression transmembranaire résulte donc d'une surpression dans le compartiment 2 et non plus d'une dépression dans le compartiment 3, le circuit de liquide usé ne comprenant pas de pompe d'extraction.

Par ailleurs, les réservoirs 11, 20 sont suspendus à deux balances indépendantes 31, 32. La mesure de la pression transmembranaire dans l'échangeur 1 peut donc se faire indirectement par une mesure de débit d'ultrafiltration au moyen des balances 31, 32 (balance 21 seule en mode hémofiltration, balances 31, 32 en mode hémodiafiltration) et il n'est pas nécessaire, pour déterminer cette pression transmembranaire, de mesurer les pressions dans chacun des compartiments 2, 3 de l'échangeur 1. Pour cette raison, les valeurs de seuil associées automatiquement par l'unité de commande 24 à la consigne de débit de sang Q_{B} et qui servent de référence pour la bascule d'un mode de traitement à l'autre, sont des valeurs de pression transmembranaire TMP₁ et TMP₂ prises comme référence dans le rein artificiel représenté sur la figure 1.

Dernière différence avec le rein précédent, dans ce second rein, c'est la pompe de circulation 13 de liquide stérile qui est asservie à la comparaison, par l'unité de commande 24, entre le débit de perte de poids souhaité Q_{WL} et le débit de perte de poids réel calculé à partir des iinformations fournies par les balances 31, 32, l'organe de retreint 30 étant réglé indépendamment par ailleurs.

Le rein représenté sur la figure 2 fonctionne de la façon suivante : lorsque le débit d'ultrafiltration atteint Q_{UF1}, l'alternance des modes hémodiafiltration/hémofiltration est commandée en fonction d'une séquence temporelle. A cette fin, deux valeurs de durée T₁ et T₂ correspondant respectivement aux deux modes de traitement sont mises initialement en mémoire dans l'unité de commande 24, le médecin pouvant, par le choix judicieux de ces valeurs ainsi que de leur ratio, obtenir pour chaque patient un rendement épuratif optimal.

La présente invention n'est pas limitée au mode de réalisation qui viennent d'être décrits et elle est susceptible de variantes.

Ainsi, la source de liquide stérile peut être constituée par une poche de solution préconditionnée ou par un conteneur prévu pour être rempli par un liquide préparé extemporanément. Le rein artificiel peut aussi comporter plusieurs sources de liquides indépendantes reliées respectivement, par l'intermédiaire des canalisations 14, 15, 16, au second compartiment 3 de l'échangeur 1 et aux pièges à bulles 8, 9.

Par ailleurs, ce liquide peut être une solution de dialyse classique, contenant tous les électrolytes du sang, ou bien une solution dépourvue d'agent tampon (bicarbonate de sodium). Dans ce dernier cas, une perfusion d'agent tampon permet de compenser les pertes diffusives qui se produisent dans l'échangeur.

Dans le mode de réalisation de la figure 2, l'organe de rétreint 30 pourrait être remplacé par une pompe pilotée par l'unité de commande 24 en fonction de la pression régnant dans le compartiment 2 de l'échangeur 1. En effet, comme, dans ce mode de réalisation particulier, la pression dans le compartiment 3 ne dépend que de la position respective des réservoirs 11, 20 et des pertes de charges dans les canalisations, cette pression dans le compartiment 2 est donc représentative de la pression transmembranaire dans l'échangeur. Dans ce cas, le débit de la pompe 13 ne serait pas régulé, mais réglé initialement à une valeur donnée.

Par ailleurs, le rein artificiel représenté sur la figure 1, peut aussi fonctionner selon le mode adopté pour le rein représenté sur la figure 2, où l'alternance des traitements par hémofiltration et hémodiafiltration est commandée en fonction d'une séquence temporelle. Les capteurs de pression 25, 26 sont alors utilisés pour vérifier que les pressions de part et d'autre de la membrane ne dépassent pas des seuils de sécurité.

En outre, bien que le fonctionnement du rein selon l'invention ait été décrit pour la mise en oeuvre d'un traitement par hémodiafiltration et d'un traitement par hémofiltration avec perfusion d'un liquide de substitution dans le circuit de circulation extracorporelle de sang en aval de l'échangeur, ce rein permet la mise en oeuvre d'autres types de traitement ; ultrafiltration pure, les clamps 17, 18, 19 étant fermés et la pompe de liquide stérile 13 étant arrêtée ; hémofiltration avec perfusion du liquide de substitution dans le circuit de circulation extracorporelle de sang, en aval de l'échangeur 1, les clamps 17, 19 étant fermés et le clamp 18 ouvert.

## Revendications

1. Rein artificiel comprenant :
- un circuit (5, 7) pour circulation extracorporelle de sang connectable à une source (11) de liquide stérile,
- un échangeur (1) ayant deux compartiments (2, 3) séparés par une membrane semi-perméable (4), un premier compartiment (2) étant connecté au circuit (5, 7) pour circulation extracorporelle de sang, un second compartiment (3) ayant une entrée connectable à une source (11) de liquide stérile,
- des moyens (22 ; 30) pour créer une pression transmembranaire positive entre le premier et le second compartiment (2, 3) de l'échangeur (1),
caractérisé en ce qu'il comprend des moyens (25, 26 ; 31, 32) pour mesurer une valeur représentative de la pression transmembranaire dans l'échangeur (1) et des moyens de commande (24) pour commander la connexion d'une source (11) de liquide stérile à l'entrée du second compartiment (3) de l'échangeur (1) quand cette valeur mesurée est supérieure à une valeur de seuil prédéterminée (TMP₁; Q_{UF1}).

2. Rein artificiel selon la revendication 1, caractérisé en ce que les moyens de commande (24) sont prévus en outre pour commander la connexion d'une source (11) de liquide stérile au circuit (5, 7) pour circulation extracorporelle de sang quand la valeur mesurée est inférieure à une deuxième valeur de seuil prédéterminée (TMP₂ ; Q_{UF2}), inférieure à la première valeur de seuil (TMP₁; Q_{UF1}).

3. Rein artificiel selon une des revendications 1 ou 2, caractérisé en ce que les moyens de commande (24) sont prévus en outre pour commander alternativement, selon une séquence temporelle, la connexion d'une source (11) de liquide stérile à l'entrée du second compartiment (3) de l'échangeur (1) et la connexion d'une source (11) de liquide stérile au circuit pour circulation extracorporelle de sang (5, 7).

4. Rein artificiel selon une des revendications 1 à 3, caractérisé en ce que les moyens pour mesurer une valeur représentative de la pression transmembranaire dans l'échangeur (1) sont des capteurs de pression (25, 26) reliés respectivement aux deux compartiments (2, 3) de l'échangeur (1).

5. Rein artificiel selon des revendications 1 à 3, caractérisé en ce que les moyens pour mesurer une valeur représentative de la pression transmembranaire dans l'échangeur (1) sont des balances (31, 32) pour peser respectivement le liquide stérile s'écoulant dans l'échangeur (1) ou dans le circuit (5, 7) pour circulation extracorporelle de sang et le liquide s'écoulant de l'échangeur (1), cette valeur étant le débit d'ultrafiltration calculé à partir des informations fournies par les balances.

6. Rein artificiel selon une des revendications 1 à 5, caractérisé en ce qu'une source de liquide stérile (11) est connectable au circuit (5, 7) pour circulation extracorporelle de sang soit en amont, soit en aval de l'échangeur (1).

7. Rein artificiel selon des revendications 1 à 6, caractérisé en ce que les moyens pour créer une pression transmembranaire dans l'échangeur (1) comprennent une pompe (22) placée sur une canalisation (21) connectée à une sortie du second compartiment (3) de l'échangeur (1).

8. Rein artificiel selon des revendications 1 à 7, caractérisé en ce qu'il comprend des moyens (23 ; 31, 32) pour faire un bilan entre le liquide stérile s'écoulant de la source de liquide (11) et le liquide usé s'écoulant du second compartiment (3) de l'échangeur (1).

9. Rein artificiel selon la revendication 8, caractérisé en ce que la source de liquide stérile est constituée par un réservoir (11) et en ce que les moyens pour faire le bilan de l'écoulement des liquides stérile et usé comprennent une balance (23) pour peser le réservoir (11) de liquide stérile ainsi qu'un second réservoir (20) pour le recueil de liquide usé.

10. Rein artificiel selon une des revendications 8 et 9, caractérisé en ce que l'unité de commande (24) est prévue pour piloter les moyens (22) pour créer une pression transmembranaire dans l'échangeur (1) en fonction de la comparaison entre la différence mesurée et la différence souhaitée entre les écoulements des liquides stérile et usé.

11. Rein artificiel selon une des revendications 1 à 10, caractérisé en ce que le circuit (5, 7) pour circulation extracorporelle de sang et le second compartiment (3) de l'échangeur (1) sont connectables à la même source de liquide (11).

12. Procédé de commande d'un rein artificiel comprenant :
- un circuit (5, 7) pour circulation extracorporelle de sang connectable à une source (11) de liquide stérile,
- un échangeur (1) ayant deux compartiments (2, 3) séparés par une membrane semi-perméable (4), un premier compartiment (2) étant connecté au circuit (5, 7) pour circulation extracorporelle de sang, un second compartiment (3) ayant une entrée connectable à une source (11) de liquide stérile,
- des moyens (22 ; 30) pour créer une pression transmembranaire positive entre le premier et le second compartiment (2, 3) de l'échangeur (1),
caractérisé en ce qu'il consiste à :
- mesurer une valeur représentative de la pression transmembranaire dans l'échangeur (1),
- comparer cette valeur à une valeur de seuil prédéterminée (TMP₁ ; Q_{UF1}), et
- commander la connexion d'une source (11) de liquide stérile à l'entrée du second compartiment (3) de l'échangeur (1) quand la valeur mesurée est supérieure à la valeur de seuil prédéterminée (TMP₁ ; Q_{UF1}).

13. Procédé de commande selon la revendication 12, caractérisé en ce qu'il consiste en outre à :
- comparer la valeur mesurée représentative de la pression transmembranaire à une deuxième valeur de seuil prédéterminée (TMP₂ ; Q_{UF2}), cette deuxième valeur de seuil étant inférieure à la première valeur de seuil (TMP₁ ; Q_{UF1}), et
- commander la connexion d'une source (11) de liquide stérile au circuit (5, 7) pour circulation extracorporelle de sang quand la valeur mesurée est inférieure à la deuxième valeur de seuil (TMP₂ ; Q_{UF2}).

14. Procédé de commande selon la revendication 12, caractérisé en ce qu'il consiste en outre, à partir du moment où la valeur mesurée a atteint la valeur de seuil prédéterminée (TMP₁ ; Q_{UF1}), à commander alternativement, selon une séquence temporelle prédéterminée (T₁, T₂), la connexion d'une source de liquide (11) à l'entrée du second compartiment (3) de l'échangeur (1) et au circuit (5, 7) pour circulation extracorporelle de sang.

15. Procédé de commande selon une des revendications 11 à 13, pour un rein artificiel comprenant en outre des moyens (23 ; 31, 32) pour faire un bilan entre le liquide stérile s'écoulant de la source de liquide (11) et le liquide usé s'écoulant du second compartiment (3) de l'échangeur (1),
caractérisé en ce qu'il consiste en outre à piloter les moyens (22) pour créer une pression transmembranaire dans l'échangeur (1) en fonction de la comparaison entre la différence mesurée et la différence souhaitée entre les écoulements des liquides stérile et usé.

## Claims

1. An artificial kidney comprising:
· a circuit (5, 7) for conveying a flow of blood outside the body connectable to a source (11) of sterile liquid;
· an exchanger (1) having two compartments (2, 3) separated by a semipermeable membrane (4), a first compartment (2) being connected to the circuit (5, 7) for conveying a flow of blood outside the body, a second compartment (3) having an inlet connectable to a source (11) of sterile liquid;
· means (22; 30) for establishing a positive transmembrane pressure between the first and second compartments (2, 3) of the exchanger (1);
the kidney being characterised in that it includes means (25, 26; 31, 32) for measuring a value representative of the transmembrane pressure in the exchanger (1), and control means (24) for causing the source (11) of sterile liquid to be connected to the inlet of the second compartment (3) of the exchanger (1) when said measured value is greater than a predetermined threshold value (TMP₁; Q_{UF1}).

2. An artificial kidney according to claim 1, characterised in that the control means (24) are also designed to connect the source (11) of sterile liquid to the circuit (5, 7) for conveying a flow of blood outside the body when the measured value is less than a second predetermined threshold value (TMP₂; Q_{UF2}), which is lower than the first threshold value (TMP₁; Q_{UF1}).

3. An artificial kidney according to claim 1 or 2, characterised in that the control means (24) are also designed to alternate in a timing sequence between connecting the source (11) of sterile liquid to the inlet of the second compartment (3) of the exchanger (1) and connecting the source (11) of sterile liquid to the circuit (5, 7) for conveying a flow of blood outside the body.

4. An artificial kidney according to any one of claims 1 to 3, characterised in that the means for measuring a value representative of the transmembrane pressure in the exchanger (1) are pressure sensors (25, 26) connected to respective ones of the two compartments (2, 3) of the exchanger (1).

5. An artificial kidney according to any one of claims 1 to 3, characterised in that the means for measuring a value representative of the transmembrane pressure in the exchanger (1) are scales (31, 32) for weighing respectively the sterile liquid flowing into the exchanger (1) or into the circuit (5, 7) for conveying a flow of blood outside the body, and the liquid flowing out from the exchanger (1), said value being the ultrafiltration flow rate calculated on the basis of the data provided by the scales.

6. An artificial kidney according to any one of claims 1 to 5, characterised in that the source (11) of sterile liquid is connectable to the circuit (5, 7) for conveying a flow of blood outside the body, either upstream or downstream from the exchanger (1).

7. An artificial kidney according to any one of claims 1 to 6, characterised in that the means for establishing a transmembrane pressure in the exchanger (1) comprise a pump (22) placed on a duct (21) connected to an outlet of the second compartment (3) of the exchanger (1).

8. An artificial kidney according to any one of claims 1 to 7, characterised in that it includes means (23; 31, 32) for measuring the difference between the flow of sterile liquid flowing out from the source of liquid (11) and the flow of waste liquid flowing out from the second compartment (3) of the exchanger (1).

9. An artificial kidney according to claim 8, characterised in that the source of sterile liquid is constituted by a container (11) and in that the means for measuring the difference between the flows of sterile liquid and waste liquid comprise scales (23) for weighing the container (11) of sterile liquid and a second container (20) for collecting the waste liquid.

10. An artificial kidney according to claim 8 or 9, characterised in that the control unit (24) is designed to control the means (22) for establishing a transmembrane pressure in the exchanger (1) as a function of a comparison between the measured difference and the desired difference between the flows of sterile liquid and waste liquid.

11. An artificial kidney according to one of the claims 1 to 10, characterised in that the circuit (5, 7) for conveying blood outside of the body and the second compartment (3) of the exchanger (1) are connectable to the same source of liquid (11).

12. A control method of controlling an artificial kidney comprising:
· a circuit (5, 7) for conveying a flow of blood outside the body connectable to a source (11) of sterile liquid;
· an exchanger (1) having two compartments (2, 3) separated by a semipermeable membrane (4), a first compartment (2) being connected to the circuit (5, 7) for conveying a flow of blood outside the body, a second compartment (3) having an inlet connectable to a source (11) of sterile liquid;
· means (22; 30) for establishing a positive transmembrane pressure between the first and second compartments (2, 3) of the exchanger (1);
the method being characterised in that it consists in:
· measuring a value representative of the transmembrane pressure in the exchanger (1),
· comparing said value with a predetermined threshold value (TMP₁; Q_{UF1}); and
· causing the source (11) of sterile liquid to be connected to the inlet of the second compartment (3) of the exchanger (1) when the measured value is greater than the predetermined threshold value (TMP₁; Q_{UF1}).

13. A control method according to claim 12, characterised in that it further consists in:
· comparing the measured value representative of the transmembrane pressure with a second predetermined threshold value (TMP₂; QUF₂), said second threshold value being less than the first threshold value (TMP₁; Q_{UF1}); and
· causing the source (11) of sterile liquid to be connected to the circuit (5, 7) for conveying a flow of blood outside the body when the measured value is less than the second threshold value (TMP₂; Q_{UF2}).

14. A control method according to claim 12, characterised in that it further consists, as from the time when the measured value reaches the predetermined threshold value (TMP₁; Q_{UF1}), in alternately controlling, in a determined timing sequence (T₁, T₂), the connection of a source of liquid (11) to the inlet of the second compartment (3) of the exchanger (1) and to the circuit (5, 7) for conveying a flow of blood outside the body.

15. A control method according to one of the claims 11 to 13, for an artificial kidney further comprising means (23; 31, 32) for measuring the difference between the flow sterile liquid flowing from the source of liquid (11) and the flow of waste liquid flowing from the second compartment (3) of the exchanger (1),
the method being characterised in that it further consists in controlling the means (22) for establishing a transmembrane pressure in the exchanger (1) as a function of the comparison between the measured difference and the desired difference between the flows of sterile liquid and of waste liquid.

## Patentansprüche

1. Künstliche Niere mit
· einem extrakorporellen Blutkreislauf (5, 7), der an eine Quelle (11) für eine sterile Flüssigkeit anschließbar ist,
· einem Austauscher (1), der zwei Teilräume (2, 3) hat, die durch eine halbdurchlässige Membran (4) getrennt sind, wobei ein erster Teilraum (2) an den extrakorporellen Blutkreislauf (5, 7) angeschlossen ist, wobei ein zweiter Teilraum (3) einen Eingang hat, der an eine Quelle (11) für sterile Flüssigkeit anschließbar ist,
· einer Einrichtung (22, 30) zum Erzeugen eines positiven Membranübergangsdrucks zwischen dem ersten und dem zweiten Teilraum (2, 3) des Austauschers (1),
dadurch gekennzeichnet, daß sie eine Einrichtung (25, 26; 31, 32) zum Messen eines Werts umfaßt, der für den Membranübergangsdruck in dem Austauscher (1) repräsentativ ist, und eine Steuereinrichtung (24) zum Steuern der Verbindung einer Quelle (11) für sterile Flüssigkeit mit dem Eingang des zweiten Teilraums (3) des Austauschers (1), wenn dieser gemessene Wert höher ist als ein vorbestimmter Sollwert (TMP₁; Q_{UF1}).

2. Künstliche Niere nach Anspruch 1, dadurch gekennzeichnet, daß die Steuereinrichtung (24) außerdem zum Steuern der Verbindung einer Quelle (11) für sterile Flüssigkeit mit dem extrakorporellen Blutkreislauf (5, 7) vorgesehen ist, wenn der gemessene Wert niedriger ist als ein zweiter vorbestimmter Sollwert (TMP₂; Q_{UF2}) der niedriger ist als der erste Sollwert (TMP₁; Q_{UF1}).

3. Künstliche Niere nach Anspruch 1 oder 2, dadurch gekennzeichnet, daß die Steuereinrichtung (24) außerdem zum abwechselnden Steuern der Verbindung einer Quelle (11) für sterile Flüssigkeit mit dem Eingang des zweiten Teilraums (3) des Austauschers (1) und der Verbindung einer Quelle (11) für sterile Flüssigkeit mit dem extrakorporellen Blutkreislauf (5, 7) gemäß einer zeitlichen Abfolge vorgesehen ist.

4. Künstliche Niere nach einem der Ansprüche 1 bis 3, dadurch gekennzeichnet, daß die Einrichtung zum Messen eines für den Membranübergangsdruck im Austauscher (1) repräsentativen Werts aus Druckfühlern (25, 26) besteht, die jeweils mit den beiden Teilräumen (2, 3) des Austauschers (1) verbunden sind.

5. Künstliche Niere nach den Ansprüchen 1 bis 3, dadurch gekennzeichnet, daß die Einrichtung zum Messen eines für den Membranübergangsdruck im Austauscher (1) repräsentativen Werts aus Waagen (31, 32) zum jeweiligen Wiegen der sterilen Flüssigkeit besteht, die in dem Austauscher (1) oder dem extrakorporellen Blutkreislauf (5, 7) strömt sowie der Flüssigkeit, die aus dem Austauscher (1) herausströmt, wobei dieser Wert der Ultrafiltrationsdurchsatz ist, der aus Informationen berechnet wird, die durch die Waagen geliefert werden.

6. Künstliche Niere nach einem der Ansprüche 1 bis 5, dadurch gekennzeichnet, daß eine Quelle (11) für sterile Flüssigkeit an den extrakorporellen Blutkreislauf (5, 7) entweder stromaufwärts oder stromabwärts vom Austauscher (1) anschließbar ist.

7. Künstliche Niere nach den Ansprüchen 1 bis 6, dadurch gekennzeichnet, daß die Einrichtung zum Erzeugen eines Membranübergangsdrucks in dem Austauscher (1) eine Pumpe (22) umfaßt, die in einer Leitung (21) angeordnet ist, die an einen Ausgang des zweiten Teilraums (3) des Austauschers (1) angeschlossen ist.

8. Künstliche Niere nach einem der Ansprüche 1 bis 7, dadurch gekennzeichnet, daß sie eine Einrichtung (23; 31, 32) zum Ziehen einer Bilanz zwischen der sterilen Flüssigkeit umfaßt, die aus der Flüssigkeitsquelle (11) strömt und der gebrauchten Flüssigkeit, die im zweiten Teilraum (3) des Austauschers (1) strömt.

9. Künstliche Niere nach Anspruch 8, dadurch gekennzeichnet, daß die Quelle für sterile Flüssigkeit durch einen Vorratsbehälter (11) gebildet ist, und daß die Einrichtung zum Ziehen der Bilanz der Strömung der sterilen und gebrauchten Flüssigkeiten eine Waage (23) zum Wiegen des Vorratsbehälters (11) für die sterile Flüssigkeit sowie einen zweiten Vorratsbehälter (20) zum Sammeln der gebrauchten Flüssigkeit umfaßt.

10. Künstliche Niere nach Anspruch 8 oder 9, dadurch gekennzeichnet, daß die Steuereinheit (24) zum Steuern der Einrichtung (22) zum Erzeugen eines Membranübergangsdrucks im Austauscher (1) als Funktion des Vergleichs zwischen der gemessenen Differenz und der gewünschten Differenz zwischen den Strömen der sterilen und gebrauchten Flüssigkeiten vorgesehen ist.

11. Künstliche Niere nach einem der Ansprüche 1 bis 10, dadurch gekennzeichnet, daß der extrakorporelle Blutkreislauf (5, 7) und der zweite Teilraum (3) des Austauschers (1) an dieselbe Flüssigkeitsquelle (11) anschließbar sind.

12. Verfahren zum Steuern einer künstlichen Niere mit
· einem extrakorporellen Blutkreislauf (5, 7), der an eine Quelle (11) für eine sterile Flüssigkeit anschließbar ist,
· einem Austauscher (1), der zwei Teilräume (2, 3) hat, die durch eine halbdurchlässige Membran (4) getrennt sind, wobei ein erster Teilraum (2) an den extrakorporellen Blutkreislauf (5, 7) angeschlossen ist, wobei ein zweiter Teilraum (3) einen Eingang hat, der an eine Quelle (11) für sterile Flüssigkeit anschließbar ist,
· einer Einrichtung (22, 30) zum Erzeugen eines positiven Membranübergangsdrucks zwischen dem ersten und dem zweiten Teilraum (2, 3) des Austauschers (1),
dadurch gekennzeichnet, daß es besteht aus :
· Messen eines Werts, der für den Membranübergangsdruck in dem Austauscher (1) repräsentativ ist,
· Vergleichen dieses Werts mit einem vorbestimmten Sollwert (TMP₁; Q_{UF1}) und
· Steuern der Verbindung einer Quelle (11) für sterile Flüssigkeit mit dem Eingang des zweiten Teilraums (3) des Austauschers (1), wenn der gemessene Wert höher als der vorbestimmte Sollwert (TMP₁; Q_{UF1}) ist.

13. Steuerungsverfahren nach Anspruch 12, dadurch gekennzeichnet, daß es außerdem besteht aus :
· Vergleichen des für den Membranübergangsdruck repräsentativen Werts mit einem zweiten vorbestimmten Sollwert (TMP₂; Q_{UF2}), wobei dieser zweite Sollwert niedriger ist als der erste Sollwert (TMP₁; Q_{UF1}), und
· Steuern der Verbindung einer Quelle (11) für sterile Flüssigkeit mit dem extrakorporellen Blutkreislauf (5, 7), wenn der gemessene Wert niedriger ist als der zweite Sollwert (TMP₂; Q_{UF2}).

14. Steuerungsverfahren nach Anspruch 12, dadurch gekennzeichnet, daß es außerdem besteht aus wechselweise Steuern der Verbindung einer Flüssigkeitsquelle (11) mit dem Eingang des zweiten Teilraums (3) des Austauschers (1) und mit dem extrakorporellen Blutkreislauf (5, 7) gemäß einem vorbestimmten zeitlichen Ablauf (T₁, T₂) beginnend mit dem Augenblick, wenn der gemessene Wert den vorbestimmten Sollwert (TMP₁; Q_{UF1}) erreicht.

15. Steuerungsverfahren nach einem der Ansprüche 11 bis 13 für eine künstliche Niere, die außerdem eine Einrichtung (23; 31, 32) zum Bilanzziehen zwischen der sterilen Flüssigkeit, die aus der Flüssigkeitsquelle (11) strömt und der gebrauchten Flüssigkeit, die aus dem zweiten Teilraum (3) des Austauschers (1) strömt umfaßt, dadurch gekennzeichnet, daß es außerdem darin besteht, die Einrichtung (22) zum Erzeugen eines Membranübergangsdrucks in dem Austauscher (1) als Funktion des Vergleichs zwischen der gemessenen Differenz und der gewünschten Differenz zwischen den Strömen der sterilen und gebrauchten Flüssigkeiten zu steuern.
